# EUROPEAN PATENT SPECIFICATION

(11) **EP 2 512 423 B1**
(45) Date of publication and mention of the grant of the patent: **12.12.2018**
(21) Application number: 09801565.4
(22) Date of filing: 18.12.2009
(51) Int. Cl.: A61K 8/41, A61Q 5/04, A61Q 5/12

(54) **PROCESS FOR TREATING KERATIN FIBERS**
VERFAHREN ZUR BEHANDLUNG VON KERATINFASERN
PROCÉDÉ DE TRAITEMENT DE FIBRES DE KÉRATINE

(43) Date of publication of application: 24.10.2012
(73) Proprietor: L'Oréal, 75008 Paris (FR)
(72) Inventor: DE BONI, Maxime, Kawasaki-shi Kanagawa 213-0012 (JP); TAKAHASHI, Hiroshi, Kawasaki-shi Kanagawa 213-0012 (JP)
(74) Representative: Lavoix
(86) International application number: PCT/JP2009/071727
(87) International publication number: WO 2011/074137

(56) References cited:
- EP-A2- 2 111 852
- WO-A1-00/64411
- JP-A- 2007 039 410
- US-A- 4 658 839
- US-A- 5 110 318
- US-A1- 2002 157 682
- US-A1- 2003 059 459
- US-A1- 2007 060 489
- US-A1- 2009 178 210
- US-A1- 2009 283 106

## Description

### TECHNICAL FIELD

The present invention relates to a process for treating keratin fibers such as hair, as well as a composition and a kit to be used for the process.

### BACKGROUND ART

Due to the many physical stresses (UV, shampoo, brushing, and the like) and chemical stresses (coloration, perm, relaxing, pollution, and the like) that keratin fibers such as hair must undergo daily, research for effectively repairing damaged keratin fibers has become important in the cosmetic treatments for keratin fibers.

Repairing damaged keratin fibers is only worthwhile if a real sensation of return to the original state of the keratin fibers is perceived. Furthermore, the repairing treatment should be effective against various stresses, as mentioned before.

Technologies (compositions and/or processes) known as treatments for repairing damaged keratin fibers that have been proposed are still insufficient insofar as they are often temporary and do not achieve proper recovery of keratin fiber integrity.

Cationic surfactants are widely used in skin care products. For example, please refer to US-A-2008-313820.

JP 2007-039410 discloses a hair treatment process comprising the steps of: pre-treating hair with an acid; conditioning the hair with Polyquaternium-11 and a hydrogenated castor oil/sebacic acid copolymer; winding hair around a rod; heating the rod; and post-treating the hair with shampooing and rinsing, wherein the heating step is not performed in an occlusive condition, and wherein the hair is completely dried by heating step (d).

### DISCLOSURE OF INVENTION

Cationic surfactants are known to be effective materials for keratin fibers, and more particularly damaged hair. Their cationic groups can easily interact with anionic moieties of keratin fibers and their hydrophobic tails confer smoothness, softness, and hydrophobic cover.

Nevertheless, their benefits on keratin fibers are still insufficient due to their low deposition yield onto the keratin fibers and low penetration into the keratin fibers. This means that the efficiency of the cosmetic treatment of keratin fibers using a cationic surfactant is low and a large amount of cationic surfactant is necessary in the formulation used for the cosmetic treatment.

Thus, an objective of the present invention is to provide a new treatment process for keratin fibers such as hair, using a cationic surfactant, even with a relatively small amount thereof, which provides the keratin fibers with good cosmetic effects, in particular superior repairing or recovering effects, which can be effective against various stresses for a long time.

The above objective of the present invention can be achieved by a process for treating keratin fibers according to claim 1 comprising the steps of: providing the keratin fibers with mechanical tension,applying onto the keratin fibers a composition comprising at least one cationic surfactant in an amount of 0.01 to 15% by weight relative to the total weight of the composition;then placing the keratin fibers in an occlusive space to restrict the evaporation of evaporable components in the composition; and then heating the keratin fibers at a temperature ranging from 60 to 200°C, wherein
the composition contains neither a reducing agent nor a source of carbonate ions of formula: 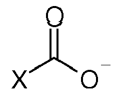 wherein
X is a group selected from the group consisting of O⁻, OH, NH₂, O-OH, and O-COO, said process further comprising the step of rinsing the keratin fibers after the step of applying the composition onto the keratin fibers and/or after the step of heating the keratin fibers.

The mechanical tension may be provided by using at least one reshaping means selected from the group consisting of a curler, a roller, a plate and an iron.

The occlusive space may be formed by at least one coating means. The coating means may be rigid or flexible. The coating means may comprise at least one member selected from the group consisting of a film and a sheet.

The keratin fibers may be heated by at least one heater providing at least one selected from the group consisting of hot air, hot steam, high frequency induction heating, microwave heating, infrared ray irradiation, laser, and flash lamp irradiation. The coating means and/or the reshaping means may comprise the heater.

The cationic surfactant may be selected from the group consisting of optionally polyoxyalkylenated primary, secondary and tertiary fatty amines, quaternary ammonium salts, and mixtures thereof.

The quaternary ammonium salts may be chosen from:
those of the general formula (V) below: 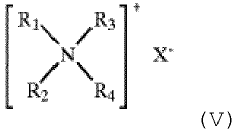 wherein
R₁, R₂, R₃, and R₄, which may be identical or different, are chosen from linear and branched aliphatic radicals comprising from 1 to 30 carbon atoms, and aromatic radicals; and X⁻ is chosen from halides, phosphates, acetates, lactates, (C₂-C₆) alkyl sulfates, and alkyl- or alkylaryl-sulfonates;
quaternary ammonium salts of imidazoline;
diquaternary ammonium salts of formula (VII): 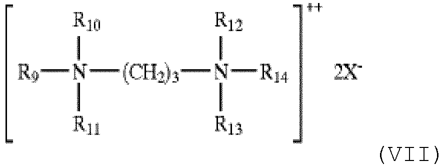 wherein R₉ is chosen from aliphatic radicals comprising from 16 to 30 carbon atoms; R₁₀, R₁₁, R₁₂, R₁₃, and R₁₄, which may be identical or different, are chosen from hydrogen and X⁻ alkyl radicals comprising from 1 to 4 carbon atoms; and X⁻ is chosen from halides, acetates, phosphates, nitrates, ethyl sulfates, and methyl sulfates; and
quaternary ammonium salts comprising at least one ester function.

The quaternary ammonium salts of imidazoline may be chosen from those of formula (VI) below: 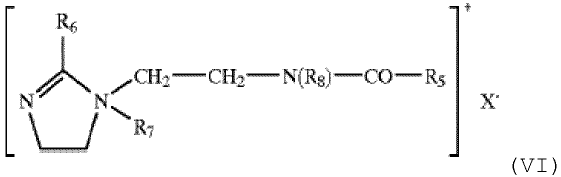 wherein
R₅ is chosen from alkenyl and alkyl radicals comprising from 8 to 30 carbon atoms; R₆ is chosen from hydrogen, C₁-C₄ alkyl radicals, and alkenyl and alkyl radicals comprising from 8 to 30 carbon atoms; R₇ is chosen from C₁-C₄ alkyl radicals; R₈ is chosen from hydrogen and C₁-C₄ alkyl radicals; and X⁻ is chosen from halides, phosphates, acetates, lactates, alkyl sulfates, alkyl sulfonates, and alkylaryl sulfonates.

The cationic surfactant may be selected from the group consisting of behenyltrimethylammonium chloride, cetyltrimethylammonium chloride, quaternium-83, quaternium-87, quaternium-22, behenylamidopropyl-2,3-di-hydroxypropyldimethylammonium chloride, palmitylamidopropyltrimethylammonium chloride, stearamidopropyldimethylamine, and chloride and methyl sulfate of diacyloxyethyl-dimethylammonium, of diacyloxyethyl-hydroxyethyl-methylamm- onium, of monoacyloxyethyl-dihydroxyethyl-methylammonium, of triacyloxyethyl-methylammonium, of monoacyloxyethyl-hydroxyethyl-dimethyl- ammonium, and mixtures thereof.

The composition comprises the cationic surfactant in an amount of 0.01 to 15% by weight relative to the total weight of the composition.

The pH of the composition may range from 4 to 11.

Further described is a composition for treating keratin fibers to be heated in an occlusive space, comprising at least one cationic surfactant,
wherein
the composition contains neither a reducing agent nor a source of carbonate ions of formula: 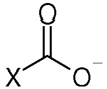 wherein
X is a group selected from the group consisting of O⁻, OH, NH₂, O-OH, and O-COO⁻.

Further described is a kit for treating keratin fibers, comprising:
a device comprising
at least one coating means to form an occlusive space, and
at least one heater to heat the keratin fibers in the occlusive space;
   and
a composition comprising at least one cationic surfactant,
   wherein
the composition contains neither a reducing agent nor a source of carbonate ions of formula: 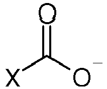 wherein
   X is a group selected from the group consisting of O⁻, OH, NH₂, O-OH, and O-COO⁻.

### BEST MODE FOR CARRYING OUT OF THE INVENTION

After diligent research, the inventors have discovered that it is possible to provide keratin fibers such as hair with good cosmetic effects, by using a cationic surfactant, and in particular superior repairing or recovering effects, which can be effective against various stresses for a long time, by using a composition comprising a cationic surfactant with a specific heating process for the keratin fibers.

The above specific heating process is performed in a closed or occlusive environment, which limits the evaporation of water or moisture from the keratin fibers and maintains the keratin fibers at higher temperature preferably in the wet state. Accordingly, the keratin fibers can be evenly heated, and the cationic surfactant can easily penetrate into or deposit onto the keratin fibers such that it can remain in or on the keratin fibers for a long time even after some stresses such as shampooing.

Since a cationic surfactant can easily stay on or in the keratin fibers, the process according to the present invention can exhibit good cosmetic effects by using even a relatively small amount of cationic surfactant as compared to a conventional process in which it is difficult for a cationic surfactant to stay on or in the keratin fibers.

The composition used in the present invention must not contain any reducing agents such as thiol-compounds. Therefore, malodor derived from the reducing agents can be prevented. Furthermore, the composition used in the present invention must not contain any carbonate ion source as defined above. Therefore, cosmetic treatment is more effective, because there is no possibility of producing carbon dioxide which may form a foam that may inhibit the deposition or penetration of the cationic surfactant on or into the keratin fibers.

### (Composition)

The composition used for the present invention comprises at least one cationic surfactant.

The cationic surfactant may be selected from the group consisting of optionally polyoxyalkylenated, primary, secondary or tertiary fatty amine salts, quaternary ammonium salts, and mixtures thereof.

Examples of quaternary ammonium salts that may be mentioned include, but are not limited to:
those of general formula (V) below: 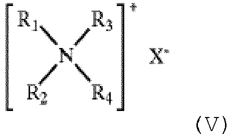 wherein
R₁, R₂, R₃, and R₄, which may be identical or different, are chosen from linear and branched aliphatic radicals comprising from 1 to 30 carbon atoms and optionally comprising heteroatoms such as oxygen, nitrogen, sulfur and halogens. The aliphatic radicals may be chosen, for example, from alkyl, alkoxy, C₂-C₆ polyoxyalkylene, alkylamide, (C₁₂-C₂₂) alkylamido (C₂-C₆) alkyl, (C₁₂-C₂₂) alkylacetate and hydroxyalkyl radicals; and aromatic radicals such as aryl and alkylaryl; and X⁻ is chosen from halides, phosphates, acetates, lactates, (C₂-C₆) alkyl sulfates and alkyl- or alkylaryl-sulfonates;
quaternary ammonium salts of imidazoline, for instance those of formula (VI) below: 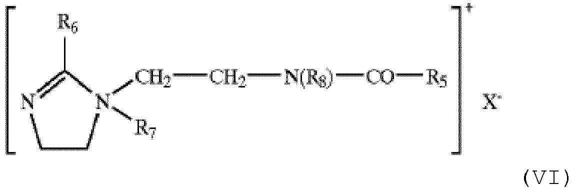 wherein:
R₅ is chosen from alkenyl and alkyl radicals comprising from 8 to 30 carbon atoms, for example fatty acid derivatives of tallow or of coconut;
R₆ is chosen from hydrogen, C₁-C₄ alkyl radicals, and alkenyl and alkyl radicals comprising from 8 to 30 carbon atoms;
R₇ is chosen from C₁-C₄ alkyl radicals;
R₈ is chosen from hydrogen and C₁-C₄ alkyl radicals; and
X⁻ is chosen from halides, phosphates, acetates, lactates, alkyl sulfates, alkyl sulfonates, and alkylaryl sulfonates. In one embodiment, R₅ and R₆ are, for example, a mixture of radicals chosen from alkenyl and alkyl radicals comprising from 12 to 21 carbon atoms, such as fatty acid derivatives of tallow, R₇ is methyl and R₈ is hydrogen. Examples of such products include, but are not limited to, Quaternium-27 (CTFA 1997) and Quaternium-83 (CTFA 1997), which are sold under the names "Rewoquat®" W75, W90, W75PG and W75HPG by the company Witco;
diquaternary ammonium salts of formula (VII): 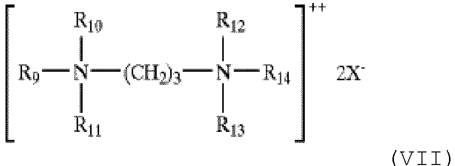 wherein:
   R₉ is chosen from aliphatic radicals comprising from 16 to 30 carbon atoms;
   R₁₀, R₁₁, R₁₂, R₁₃, and R₁₄, which may be identical or different, are chosen from hydrogen and alkyl radicals comprising from 1 to 4 carbon atoms; and
   X⁻ is chosen from halides, acetates, phosphates, nitrates, ethyl sulfates, and methyl sulfates. An example of one such diquaternary ammonium salt is propanetallowdiammonium dichloride; and
   quaternary ammonium salts comprising at least one ester function, such as those of formula (VIII) below: 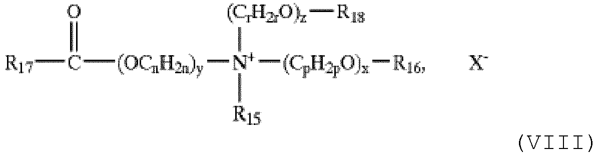 wherein:
   R₁₅ is chosen from C₁-C₆ alkyl radicals and C₁-C₆ hydroxyalkyl and dihydroxyalkyl radicals;
   R₁₆ is chosen from:
      the radical blow: 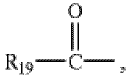
   linear and branched, saturated and unsaturated C₁-_{C22} hydrocarbon-based radicals R₂₀, and hydrogen,
   R₁₈ is chosen from:
      the radical below: 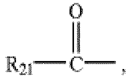
   linear and branched, saturated and unsaturated C₁-C₆ hydrocarbon-based radicals R₂₂, and hydrogen,
   R₁₇, R₁₉, and R₂₁, which may be identical or different, are chosen from linear and branched, saturated and unsaturated, C₇-C₂₁, hydrocarbon-based radicals;
   r, n, and p, which may be identical or different, are chosen from integers ranging from 2 to 6;
   y is chosen from integers ranging from 1 to 10;
   x and z, which may be identical or different, are chosen from integers ranging from 0 to 10;
   X⁻ is chosen from simple and complex, organic and inorganic anions; with the proviso that the sum x+y+z ranges from 1 to 15, that when x is 0, R₁₆ denotes R₂₀, and that when z is 0, R₁₈ denotes R₂₂. R₁₅ may be chosen from linear and branched alkyl radicals. In one embodiment, R₁₅ is chosen from linear alkyl radicals. In another embodiment, R₁₅ is chosen from methyl, ethyl, hydroxyethyl, and dihydroxypropyl radicals, for example methyl and ethyl radicals. In one embodiment, the sum x+y+z ranges from 1 to 10. When R₁₆ is a hydrocarbon-based radical R₂₀, it may be long and comprise from 12 to 22 carbon atoms, or short and comprise from 1 to 3 carbon atoms. When R₁₈ is a hydrocarbon-based radical R₂₂, it may comprise, for example, from 1 to 3 carbon atoms. By way of a non-limiting example, in one embodiment, R₁₇, R₁₉, and R₂₁, which may be identical or different, are chosen from linear and branched, saturated and unsaturated, C₁₁-C₂₁ hydrocarbon-based radicals, for example from linear and branched, saturated and unsaturated C₁₁-C₂₁ alkyl and alkenyl radicals. In another embodiment, x and z, which may be identical or different, are 0 or 1. In one embodiment, y is equal to 1. In another embodiment, r, n and p, which may be identical or different, are equal to 2 or 3, for example equal to 2. The anion X⁻ may be chosen from, for example, halides, such as chloride, bromide, and iodide; and C1-C4 alkyl sulfates, such as methyl sulfate. However, methanesulfonate, phosphate, nitrate, tosylate, an anion derived from an organic acid, such as acetate and lactate, and any other anion that is compatible with the ammonium comprising an ester function, are other non-limiting examples of anions that may be used according to the invention. In one embodiment, the anion X⁻ is chosen from chloride and methyl sulfate.

In another embodiment, the ammonium salts of formula (VIII) may be used, wherein:
R₁₅ is chosen from methyl and ethyl radicals,
x and y are equal to 1;
z is equal to 0 or 1;
r, n and p are equal to 2;
R₁₆ is chosen from:
   the radical below: 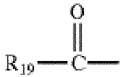
methyl, ethyl, and C₁₄-C₂₂ hydrocarbon-based radicals, hydrogen;
R₁₈ is chosen from:
   the radical below: 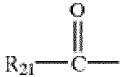 hydrogen;
R₁₇, R₁₉, and R₂₁, which may be identical or different, are chosen from linear and branched, saturated and unsaturated, C₁₃-C₁₇ hydrocarbon-based radicals, for example from linear and branched, saturated and unsaturated, C₁₃-C₁₇ alkyl and alkenyl radicals.

In one embodiment, the hydrocarbon-based radicals are linear.

Non-limiting examples of compounds of formula (VIII) that may be mentioned include salts, for example chloride and methyl sulfate, of diacyloxyethyl-dimethylammonium, of diacyloxyethyl-hydroxyethyl-methylamm- onium, of monoacyloxyethyl-dihydroxyethyl-methylammonium, of triacyloxyethyl-methylammonium, of monoacyloxyethyl-hydroxyethyl-dimethyl- ammonium, and mixtures thereof. In one embodiment, the acyl radicals may comprise from 14 to 18 carbon atoms, and may be derived, for example, from a plant oil, for instance palm oil and sunflower oil. When the compound comprises several acyl radicals, these radicals may be identical or different.

These products may be obtained, for example, by direct esterification of optionally oxyalkylenated triethanolamine, triisopropanolamine, alkyldiethanolamine or alkyldiisopropanolamine onto fatty acids or onto mixtures of fatty acids of plant or animal origin, or by transesterification of the methyl esters thereof. This esterification may be followed by a quaternization using an alkylating agent chosen from alkyl halides, for example methyl and ethyl halides; dialkyl sulfates, for example dimethyl and diethyl sulfates; methyl methanesulfonate; methyl para-toluenesulfonate; glycol chlorohydrin; and glycerol chlorohydrin.

Such compounds are sold, for example, under the names Dehyquart® by the company Cognis, Stepanquat® by the company Stepan, Noxamium® by the company Ceca, and "Rewoquat® WE 18" by the company Rewo-Goldschmidt.

The compositions according to the invention may comprise, for example, a mixture of quaternary ammonium mono-, di- and triester salts with a weight majority of diester salts.

A non-limiting example of a mixture of ammonium salts that may be used in the compositions according to the invention is that comprising from 15% to 30% by weight of acyloxyethyl-dihydroxyethyl-methylammonium methyl sulfate, from 45% to 60% by weight of diacyloxyethyl-hydroxyethyl-methylammonium methyl sulfate, and from 15% to 30% by weight of triacyloxyethyl-methylammonium methyl sulfate, the acyl radicals of all these compounds comprising from 14 to 18 carbon atoms and being derived from optionally partially hydrogenated palm oil.

Other non-limiting examples of ammonium salts that may be used in the compositions according to the invention include the ammonium salts comprising at least one ester function described in U.S. Pat. Nos. 4,874,554 and 4,137,180.

Among the quaternary ammonium salts mentioned above that may be used in compositions according to the invention include, but are not limited to, those corresponding to formula (V), for example tetraalkylammonium chlorides, for instance dialkyldimethylammonium and alkyltrimethylammonium chlorides in which the alkyl radical comprises from about 12 to 22 carbon atoms, such as behenyltrimethylammonium, distearyldimethylammonium, cetyltrimethylammonium and benzyldimethylstearylammonium chloride; palmitylamidopropyltrimethylammonium chloride; and stearamidopropyldimethyl(myristyl acetate)ammonium chloride, sold under the name "Ceraphyl® 70" by the company Van Dyk.

According to one embodiment, the at least one cationic surfactant that may be used in the compositions of the invention is chosen from quaternary ammonium salts, for example from behenyltrimethylammonium chloride, cetyltrimethylammonium chloride, Quaternium-83, Quaternium-87, Quaternium-22, behenylamidopropyl-2,3-dihydroxypropyldimethylammonium chloride, palmitylamidopropyltrimethylammonium chloride, and stearamidopropyldimethylamine.

The compositions according to the invention comprises the at least one cationic surfactant in an amount ranging from 0.01% to 15% by weight, relative to the total weight of the composition, for example from 0.1% to 10% by weight, relative to the total weight of the composition, such as from 0.2% to 5% by weight, relative to the total weight of the composition.

The composition used for the present invention contains neither a reducing agent nor a source of carbonate ions of formula: 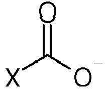 wherein
X is a group selected from the group consisting of O⁻, OH, NH₂, O-OH, and O-COO⁻.

The pH of the composition may range from 4 to 11, preferably between 4.0 and 9.0, and more preferably between 6.0 to 8.0. Since the pH of the composition is not relatively high or low, damage to the keratin fibers by the composition can be reduced. In order to adjust the pH, an acidic or alkali agent(s) other than sources of ions of the invention may be used alone or in combination. The amount of the acidic or alkali agent(s) is not limited, but may be from 0.1 to 5% by weight relative to the total weight of the composition. As the acidic agents, mention may be made of any inorganic or organic acids which are commonly used in cosmetic products such as citric acid, lactic acid, phosphoric acid or hydrochloric acid (HCl). HCl is preferable. As the alkali agents, mention may be made of any inorganic or organic basic agents which are commonly used in cosmetic products such as ammonia; alcanolamines such as mono-, di- and triethanolamine, isopropanolamine; sodium and potassium hydroxides; urea, guanidine and their derivatives; basic amino acids such as lysine or arginine; and diamines such as those described in the structure below: 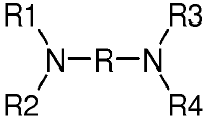 wherein R denotes an alkylene such as propylene optionally substituted by a hydroxyl or a C₁-C₄ alkyl radical, and R₁, R₂, R₃ and R₄ independently denote a hydrogen atom, an alkyl radical or a C₁-C₄ hydroxyalkyl radical, which may be exemplified by 1,3-propanediamine and derivatives thereof. Arginine and monoethanolamine are preferred.

The composition used for the present invention may also comprise one or more additional cosmetic agent(s). The amount of the additional cosmetic agent(s) is not limited, but may be from 0.1 to 10% by weight relative to the total weight of the composition. The cosmetic agent(s) may be selected from the group consisting of volatile or non volatile, linear or cyclic, amine-type or not, silicones, cationic, anionic, non ionic or amphoteric polymers, peptides and derivatives thereof, protein hydrolyzates, synthetic or natural waxes, and especially fatty alcohols, swelling agents and penetrating agents, as well as other active compounds, such as anionic, non ionic, amphoteric or zwitterionic surfactants, agents for combating hair loss, anti-dandruff agents, associative-type or not, natural or synthetic thickeners, suspending agents, sequestering agents, opacifying agents, dyes, sunscreen agents, fillers, vitamins or provitamins, mineral, vegetable or synthetic oils, as well as fragrances, preserving agents, stabilizers, and mixtures thereof.

The vehicle for the composition used for the present invention is preferably an aqueous medium consisting of water and may advantageously contain one or several cosmetically acceptable organic solvents, which particularly include alcohols, such as ethyl alcohol, isopropyl alcohol, benzyl alcohol and phenylethyl alcohol, or polyols or polyol ethers, such as ethylene glycol monomethyl, monoethyl and monobutyl ethers, propylene glycol or ethers thereof, such as propylene glycol monomethylether, butylene glycol, dipropylene glycol as well as diethylene glycol alkyl ethers, such as diethylene glycol monoethylether or monobutylether. The water may be present in a concentration of from 10 to 90% by weight relative to the total weight of the composition. The organic solvent(s) may then be present in a concentration of from 0.1 to 20% by weight, and preferably from 1 to 10% by weight relative to the total weight of the composition.

The composition used in the present invention may exist in any form such as a lotion, a gel, thickened or not, or a cream.

### (Keratin Fiber Treatment Process)

The process for treating keratin fibers according to the present invention can be performed by
applying onto the keratin fibers a composition comprising at least one cationic surfactant, as described above;
then placing the keratin fibers in an occlusive space; and then heating the keratin fibers,

According to the present invention relating to the treatment process for keratin fibers, keratin fibers such as hair are subjected to a specific heating process which is performed in an occlusive space.

The heating process can be performed by any heating means which can be freely controlled to realize the temperature desired for the process.

The heating process may preferably be performed by using a special heating device or devices that can form an occlusive space to restrict the evaporation of evaporable components such as water in the above-described composition from keratin fibers and keep a predetermined temperature in the heating device throughout the process.

If the evaporable components such as water in the above-described composition evaporate from the keratin fibers, most of the heat energy applied to the keratin fibers will be consumed by the evaporation, and therefore the temperature of the keratin fibers cannot increase up to the predetermined temperature until all evaporable components in the composition evaporate.

The above heating device may comprise a heat energy source being either in contact with keratin fibers or apart from keratin fibers, and at least one means to form an occlusive space surrounding the keratin fibers.

The heat energy source is used to heat keratin fibers. The heat energy source may be at least one heater providing at least one selected from the group consisting of hot air, hot steam, high frequency induction heating, microwave heating, infrared ray irradiation, laser, and flash lamp irradiation.

The occlusive space may be formed by at least one coating means. A plurality of coating means may be used. The coating means may be rigid or flexible.

The coating means may comprise at least one member selected from the group consisting of a film and a sheet. The material of the film or the sheet is not limited. For example, the film or the sheet may comprise a thermoplastic or thermosetting resin, a paper, a textile, a bonnet, a metal foil such as aluminum foil, and the like.

For example, the film or sheet may be set on a heating rod, a heating bar or a heating plate which is covered by keratin fibers.

According to the present invention, the coating means may comprise the heat energy source. Therefore, for example, the film or sheet which includes a heater may be set on a rod, a bar, or a plate which is covered by keratin fibers.

The occlusive conditions can restrict the evaporation of evaporable components such as water in the above-described composition applied to keratin fibers, and therefore the temperature of the keratin fibers can be increased higher than that obtainable by a conventional heating process or device for the keratin fibers in open conditions. Furthermore, the keratin fibers can be heated effectively, and the keratin fibers can be heated evenly.

According to one variant of the present invention, the occlusive space may comprise apertures, the surface area of which is less than 5%, preferably less than 3% and more particularly less than 0.5% of the total surface area of the coating means. According to this variant, the total surface area of the coating means comprises the surface area of, when it is present, an opening means for the coating means.

The apertures may be passages, holes or orifices, which may allow an exchange of air between the occlusive space and the exterior thereof, especially when the reaction such as forming vapor inside the occlusive space is too great. On the other hand, a person skilled in the art could form the apertures such that the diffusion of heat in the occlusive space is not impaired.

The keratin fibers are heated at 60ºC to 200ºC, more preferably 60ºC to 150ºC, more preferably 60ºC to 90ºC, during the step of heating the keratin fibers.

The heating process may be performed for an appropriate time which is required to treat keratin fibers. The time length for the heating process is not limited, but it may be from 1 minute to 2 hours, preferably 1 minute to 1 hour, and more preferably 1 minute to 30 minutes. For example, the time for heating may be from 5 to 20 minutes, preferably 10 to 15 minutes.

The keratin fibers are rinsed after the step of applying the composition onto the keratin fibers and/or after the step of heating the keratin fibers.

### (Permanent Deformation Process for Keratin Fiber)

According to the present invention relating to the treatment process for keratin fibers, the keratin fiber are subjected to mechanical tension which is typically used for permanent deformation.

The permanent deformation process for keratin fibers when mechanical tension is applied to keratin fibers may be performed as follows.

First, keratin fibers are subjected to mechanical tension for deformation. The mechanical tension can be applied to the keratin fibers by any means to deform the keratin fibers to an intended shape. For example, the mechanical tension may be provided by at least one reshaping means selected from the group consisting of a curler, a roller, a clip, a plate and an iron. The reshaping means may comprise at least one heater as described above. If the keratin fibers are rolled around a curler, this rolling-up may be performed on the entire length of the keratin fibers or, for example, on half the length of the keratin fibers. Depending on, for example, the desired hairstyle shape and amount of curls, the rolling-up may be performed with more or less thick locks.

Next, the above-described composition is applied to the keratin fibers. The application of the composition may be performed by any means, such as a brush and a comb. The keratin fibers to which the mechanical tension has been applied should be treated with the composition. It may be possible that the keratin fibers are left as they are for a certain amount of time, if necessary. Lastly, the above-described heating process is performed. The heat energy is applied to the keratin fibers under occlusive conditions as described above.

This process for permanent deformation of keratin fibers may be performed without any step of oxidizing the keratin fibers. Therefore, the time required for the process according to the present invention can be shorter than that for a conventional process which needs an oxidizing step. Furthermore, damage to the keratin fibers by the oxidizing step can be avoided.

The keratin fibers are rinsed after the step of applying the composition onto the keratin fibers and/or after the step of heating the keratin fibers.

One embodiment of the cosmetic treatment process according to the present invention may be a process for reshaping or permanent deforming keratin fibers, in particular hair, comprising:
a) a step of placing the keratin fibers under mechanical tension by rolling them up on at least one reshaping or mechanically tensioning means so as to form curls;
b) a step of applying the above-described composition to the keratin fibers;
c) an optional step of rinsing the keratin fibers,
d) a step of placing at least one coating means on the reshaping or mechanically tensioning means or vice versa to form one or more occlusive spaces; and
e) a step of heating the keratin fibers at a temperature of between 45 ± 2 or 3°C and 250 ± 2 or 3°C for 1 minute to 2 hours.

In this process, the temperature can be set, adjusted and regulated by using one or more heating means, and may be measured with a thermo-measurement probe such as Digital Surface Sensor Module, reference MT-144, sold by Sakaguchi E.H VOC Corp (Japan), set on the keratin fibers. Normally, the probe is set on a single keratin fiber. However, it is advantageous that the probe is set on the part of the keratin fibers which directly contacts with the occlusive space, and more preferably, the probe is set on the part of the keratin fibers which directly contacts with the occlusive space and forms the curl end of the keratin fibers, if a curler is used.

Preferably, the temperature is measured at atmospheric pressure of 101325 Pa.

According to the present invention, the temperature of the keratin fibers may be constant with a fluctuation of ± 2 or 3°C over the head, if the keratin fibers are hair, of an individual, and the probe may be set on any type of keratin fibers.

If the keratin fibers are hair, according to the present invention, the constant temperature with a fluctuation of ± 2 or 3°C can be obtained for any type of hair, and the temperature of the hair can be controlled to be constant ± 2 or 3°C during the heating of the hair at a certain temperature. Thus, the hair style becomes uniform and homogeneous for the entirety of the hair, and a more excellent hair style can be finally obtained.

Advantageously, the coating means may comprise one or more thermal insulating materials, and more advantageously, the coating means may consist of the material(s).

The term "thermal insulating material" means any material which has an electric conductivity of 0 to 1 W/m°C (PVC: 0.17 W/m°C).

Preferably, the heating means may be adjusted such that the temperature measured on the keratin fibers is 50°C or more, more preferably 55°C to less than 150°C, and further more preferably less than 100°C. It is preferable that the heating is performed by heating via electrical resistance.

Advantageously, the coating means is impermeable with regard to the composition used in the step b).

In the above embodiment, at least one of the reshaping or mechanical tensioning means and at least one of the covering means may include a heater.

In the above embodiment, "occlusive space" means that when the coating means is placed on the reshaping or mechanical tensioning means, or vice versa, they together form a closed structure in which heat can diffuse, but heat cannot diffuse out of or is difficult to diffuse out of the closed structure. It is preferable that the coating means and the reshaping or mechanical tensioning means can form the occlusive space when they are set on the head, if the keratin fibers are hair.

The occlusive space may form a condensation cage in which water and a component or components in the composition used in the step b) may evaporate from the keratin fibers, adhere to the wall of the coating means, and drop onto the keratin fibers. This cycle may be repeated during the heating of the keratin fibers. Thus, the keratin fibers can be always kept wet, and drying and deteriorating of the keratin fibers will be prevented.

The formation of occlusive space is an important characteristic of the present invention, because the keratin fibers in the occlusive space can be kept wet and the temperature of the keratin fibers can be constant.

Preferably, the process of the present invention may comprise an additional step of tightening the coating means on the head of an individual, if the keratin fibers are hair, by an elastic cord, an extensible band, or a stretch.

According to the process of the present invention, because of the occlusive space in which the composition can be continuously condensed on the keratin fibers, the amount of a cosmetic component or components in the composition is advantageously reduced as compared to the processes in the prior art. The amount of the cosmetic component(s) may be 0.3 to 3wt% of the composition.

In a preferred embodiment, a coating means may be placed on each hair curler as the reshaping or mechanically tensioning means, if the keratin fibers are hair. In other words, each of the hair curlers, if two or more hair curlers are used, may be covered individually by a coating means. It is advantageous to cover each hair curler because leaking to the scalp of the composition which has been applied onto keratin fibers in the step b) can be prevented.

In another preferred embodiment, a coating means may cover all hair curlers, if two or more hair curlers are used. In other words, the coating means may cover the entirety of the head if the keratin fibers are hair.

Advantageously, the occlusive space formed in the step d) may be maintained during the step e). In other words, the coating means may be removed only after the step e) or after the stop of the heating in the step e).

If necessary, the composition may be applied to keratin fibers before applying mechanical tension to the keratin fibers. It may be possible that the keratin fibers are left as they are for a certain amount of time, if necessary, before and/or after applying mechanical tension to the keratin fibers, before and/or after applying the above-described composition to the keratin fibers, and before and/or after heating the keratin fibers. After the above step e), if necessary, the keratin fibers may be fixed by oxidation after being taken out from the coating means.

### (Products)

Further described is a composition for treating keratin fibers to be heated in an occlusive space, comprising at least one cationic surfactant,
wherein
the composition contains neither a reducing agent nor a source of carbonate ions of formula: 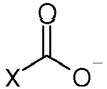 wherein
X is a group selected from the group consisting of O⁻, OH, NH₂, O-OH, and O-COO⁻.

This composition may not need to be used in combination with an oxidizing agent which is used in a conventional permanent deformation of keratin fibers. Therefore, if keratin fibers should be permanently deformed, the composition may be used in one step, whereas two steps (reducing step and oxidizing step) are necessary in the conventional permanent deformation of keratin fibers.

This composition may have the same technical features as those of the composition described above.

Further described is a kit for treating keratin fibers, comprising:
a device comprising
   at least one reshaping means to provide the keratin fibers with mechanical tension,
   at least one coating means to form an occlusive space, and
   at least one heater to heat the keratin fibers in the occlusive space;
   and
a composition comprising at least one cationic surfactant wherein the composition contains neither a reducing agent nor a source of carbonate ions of formula: 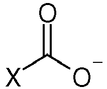 wherein
   X is a group selected from the group consisting of O⁻, OH, NH₂, O-OH, and O-COO⁻.

The coating means and the heater, as well as the composition in the kit, may be the same as those described above.

### EXAMPLES

The present invention will be described in more detail by way of examples, which however should not be construed as limiting the scope of the present invention.

### Composition 1

A hair treatment composition (referred to as "Composition 1") having the following composition shown in Table 1 was prepared (active ingredients in wt %).

**Table 1**

| | |
|---|---|
| Cetearyl alcohol | 4 |
| Propylene glycol | 2 |
| Behenyl trimethyl ammonium chloride | 2 |
| Cocamidopropyl betaine | 10 |
| Preservatives | qs |
| pH adjuster | qsp pH 6.7±0.2 |
| Water | qsp 100 |

### Example 1

Composition 1 was applied for 5 minutes on a 1g natural Japanese hair swatch previously wrapped on a 1.7 cm heating perm-roller. Then, the perm-roller was covered by a plastic film and plugged into a Digital Perm Machine (Oohiro, model ODIS-2). After a heating process at 90°C for 5 minutes, the hair was rinsed, removed from the perm-roller and dried.

The hair was soft, detangled, and smooth (smoother than the hair in Comparative Example 1).

### Comparative Example 1

Composition 1 was applied for 5 minutes on a 1g natural Japanese hair swatch which was the same as that used in Example 1. After leaving the hair without heating for 5 minutes, the hair was rinsed and dried.

The hair was slightly smoother than before the treatment. However, this slight cosmetic improvement disappeared after the first shampoo.

### Composition 2

A hair treatment composition (referred to as "Composition 2") having the following composition shown in Table 2 was prepared (active ingredients in wt %).

**Table 2**

| | |
|---|---|
| Sodium carboxymethyl starch/ethanol (96:4) | 1.5 |
| Propylene glycol | 2.5 |
| Cetrimonium chloride at 25% in water | 1 |
| Quaternium-22 at 60% in water | 0.5 |
| Preservatives | qs |
| Citric acid | qsp pH 50±.2 |
| Water | qsp 100 |

### Example 2

Composition 2 was applied for 10 minutes on a 1g Japanese hair swatch. Then, the hair was wrapped in a plastic film and covered by a flexible heating film (FTH-050 from Tokyo Technological Labo). The hair was heated in occlusive conditions for 10 minutes at 90°C. After the heating step, the hair was rinsed and dried.

The hair was very soft and smooth.

### Comparative Example 2

Composition 2 was applied for 10 minutes on a 1g Japanese hair swatch which was the same as that used in Example 2. After leaving the hair without heating for 10 minutes, the hair was rinsed and dried.

No specific change was observed on the hair after the treatment.

## Claims

1. A permanent deformation process for keratin fibers comprising the steps of:
providing the keratin fibers with mechanical tension,
applying onto the keratin fibers a composition comprising at least one cationic surfactant in an amount of 0.01 to 15% by weight relative to the total weight of the composition;
then placing the keratin fibers in an occlusive space to restrict the evaporation of evaporable components in the composition; and then heating the keratin fibers at a temperature ranging from 60 to 200°C,
wherein
the composition contains neither a reducing agent nor a source of carbonate ions of formula: 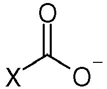 wherein
X is a group selected from the group consisting of O⁻, OH, NH₂, O-OH, and O-COO⁻,
said process further comprising the step of rinsing the keratin fibers after the step of applying the composition onto the keratin fibers and/or after the step of heating the keratin fibers.

2. The process according to Claim 1, wherein the step of providing the keratin fibers with mechanical tension is placed after the step of applying onto the keratin fibers the composition comprising at least one cationic surfactant.

3. The process according to Claim 1 or 2, wherein the occlusive space is formed by at least one coating means.

4. The process according to Claim 3, wherein the coating means is rigid or flexible.

5. The process according to Claim 3 or 4, wherein the coating means comprises at least one member selected from the group consisting of a film and a sheet.

6. The process according to any one of Claims 1 to 5, wherein the keratin fibers are heated at 60ºC to 150ºC during the step of heating the keratin fibers.

7. The process according to any one of Claims 1 to 6, wherein the keratin fibers are heated by at least one heater providing at least one selected from the group consisting of hot air, hot steam, high frequency induction heating, microwave heating, infra-red ray irradiation, laser, and flash lamp irradiation.

8. The process according to Claim 7, wherein the coating means comprises the heater.

9. The process according to any one of Claims 1 to 8, wherein the cationic surfactant is selected from the group consisting of optionally polyoxyalkylenated primary, secondary and tertiary fatty amines, quaternary ammonium salts, and mixtures thereof.

10. The process according to Claim 9, wherein the quaternary ammonium salts are chosen from:
those of the general formula (V) below: 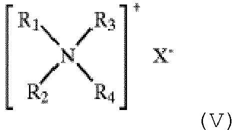 wherein
R₁, R₂, R₃, and R₄, which may be identical or different, are chosen from linear and branched aliphatic radicals comprising from 1 to 30 carbon atoms, and aromatic radicals; and X⁻ is chosen from halides, phosphates, acetates, lactates, (C₂-C₆) alkyl sulfates, and alkyl- or alkylaryl-sulfonates; quaternary ammonium salts of imidazoline;
diquaternary ammonium salts of formula (VII): 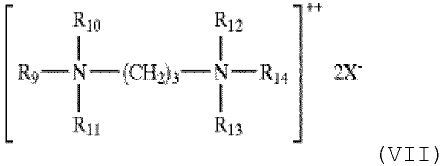 wherein R₉ is chosen from aliphatic radicals comprising from 16 to 30 carbon atoms; R₁₀, R₁₁, R₁₂, R₁₃, and R₁₄, which may be identical or different, are chosen from hydrogen and alkyl radicals comprising from 1 to 4 carbon atoms; and X⁻ is chosen from halides, acetates, phosphates, nitrates, ethyl sulfates, and methyl sulfates; and
quaternary ammonium salts comprising at least one ester function.

11. The process according to Claim 10, wherein the quaternary ammonium salts of imidazoline are chosen from those of formula (VI) below: 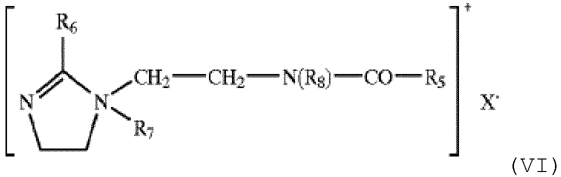 wherein
R₅ is chosen from alkenyl and alkyl radicals comprising from 8 to 30 carbon atoms; R₆ is chosen from hydrogen, C₁-C₄ alkyl radicals, and alkenyl and alkyl radicals comprising from 8 to 30 carbon atoms; R₇ is chosen from C₁-C₄ alkyl radicals; R₈ is chosen from hydrogen and C₁-C₄ alkyl radicals; and X⁻ is chosen from halides, phosphates, acetates, lactates, alkyl sulfates, alkyl sulfonates, and alkylaryl sulfonates.

12. The process according to any one of Claims 1 to 11, wherein the cationic surfactant is selected from the group consisting of behenyltrimethylammonium chloride, cetyltrimethylammonium chloride, quaternium-83, quaternium-87, quaternium-22, behenylamidopropyl-2,3-di-hydroxypropyldimethylammonium chloride, palmitylamidopropyltrimethylammonium chloride, and stearamidopropyldimethylamine.

## Patentansprüche

1. Verfahren zur permanenten Verformung von Keratinfasern, umfassend die folgenden Schritte:
Ausstatten der Keratinfasern mit mechanischer Spannung,
Anwenden einer Zusammensetzung auf die Keratinfasern, umfassend mindestens ein kationisches oberflächenaktives Mittel in einer Menge von 0,01 bis 15 Gew% mit Bezug auf das Gesamtgewicht der Zusammensetzung;
dann Anordnen der Keratinfasern in einen Okklusivraum, um das Verdunsten von verdunstbaren Bestandteilen in der Zusammensetzung zu begrenzen, und dann Erhitzen der Keratinfasern auf eine Temperatur, die im Bereich von 60 bis 200 °C liegt,
wobei
die Zusammensetzung weder ein Reduktionsmittel noch eine Quelle von Karbonationen der folgenden Formel enthält: 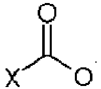 wobei
X eine Gruppe ist, ausgewählt aus der Gruppe, bestehend aus O⁻, OH, NH₂, O-OH et O-COO⁻,
wobei das Verfahren weiter den Schritt des Spülens der Keratinfasern nach dem Schritt des Anwendens der Zusammensetzung auf die Keratinfasern und/oder nach dem Schritt des Erhitzens der Keratinfasern umfasst.

2. Verfahren nach Anspruch 1, wobei der Schritt des Ausstattens der Keratinfasern mit mechanischer Spannung nach dem Schritt des Anwendens auf die Keratinfasern der Zusammensetzung, umfassend mindestens ein kationisches oberflächenaktives Mittel, angeordnet wird.

3. Verfahren nach Anspruch 1 oder 2, wobei der Okklusivraum durch mindestens ein Beschichtungsmittel gebildet ist.

4. Verfahren nach Anspruch 3, wobei das Beschichtungsmittel starr oder flexibel ist.

5. Verfahren nach Anspruch 3 oder 4, wobei das Beschichtungsmittel mindestens ein Element umfasst, ausgewählt aus der Gruppe, bestehend aus einem Film und einem Blatt.

6. Verfahren nach einem der Ansprüche 1 bis 5, wobei die Keratinfasern auf 60 ºC bis 150 ºC während des Schritt des Erhitzens der Keratinfasern erhitzt werden.

7. Verfahren nach einem der Ansprüche 1 bis 6, wobei die Keratinfasern durch mindestens eine Heizvorrichtung erhitzt werden, die mindestens eines, ausgewählt aus der Gruppe, bestehend aus heißer Luft, heißem Dampf, Hochfrequenz-Induktionserhitzung, Mikrowellenerhitzung, Infrarot-Bestrahlung, Laser und Blitzlampen-Bestrahlung bereitstellt.

8. Verfahren von Anspruch 7, wobei das Beschichtungsmittel die Heizvorrichtung umfasst.

9. Verfahren nach einem der Ansprüche 1 bis 8, wobei das kationische oberflächenaktive Mittel ausgewählt ist aus der Gruppe, bestehend aus optional polyoxyalkylenierten primären, sekundären und tertiären Fettaminen, quaternären Ammoniumsalzen und Mischungen daraus.

10. Verfahren von Anspruch 9, wobei die quaternären Ammoniumsalze ausgewählt sind aus denjenigen der allgemeinen Formel (V) unten: 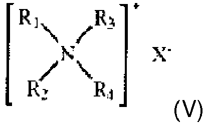 wobei
R₁, R₂, R₃ und R₄, die identisch oder verschieden sein können, ausgewählt sind aus linearen oder verzweigten aliphatischen Radikalen, umfassend von 1 bis 30 Kohlenstoffatome, und aromatisch Radikale; und X⁻ ausgewählt ist aus Haliden, Phosphaten, Acetaten, Lactaten, (C₂-C₆)-Alkylsulfaten und Alkyl- oder AlkylarylSulfonaten; quaternären Ammoniumsalzen von Imidazolin; diquaternären Ammoniumsalzen der Formel (VII): 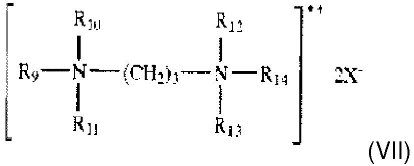 wobei R₉ ausgewählt ist aus aliphatischen Radikalen, umfassend von 16 bis 30 Kohlenstoffatome; R₁₀, R₁₁, R₁₂, R₁₃ und R₁₄, die identisch oder verschieden sein können, ausgewählt sind aus Wasserstoff und Alkylradikalen, umfassend von 1 bis 4 Kohlenstoffatome; und X⁻ ausgewählt ist aus Haliden, Acetaten, Phosphaten, Nitraten, Ethylsulfaten und Methylsulfaten;und quaternären Ammoniumsalzen, umfassend mindestens eine Esterfunktion.

11. Verfahren von Anspruch 10, wobei die quaternären Ammoniumsalze von Imidazolin ausgewählt sind aus denjenigen der allgemeinen Formel (VI) unten: 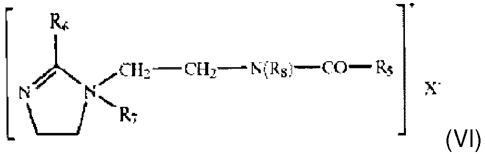 wobei
R₅ ausgewählt ist aus Alkenyl- und Alkylradikalen, umfassend von 8 bis 30 Kohlenstoffatome; R₆ ausgewählt ist aus Wasserstoff, C₁-C₄-Alkylradikalen und Alkenyl und Alkylradikalen, umfassend von 8 bis 30 Kohlenstoffatome; R₇ ausgewählt ist aus C₁-C₄-Alkylradikalen; R₈ ausgewählt ist aus Wasserstoff und C₁-C₄-Alkylradikalen; und X⁻ ausgewählt ist aus Haliden, Phosphaten, Acetaten, Lactaten, Alkylsulfaten, Alkylsulfonaten und Alkylarylsulfonaten.

12. Verfahren nach einem der Ansprüche 1 bis 11, wobei das kationische oberflächenaktive Mittel ausgewählt ist aus der Gruppe, bestehend aus Behenyltrimethylammoniumchlorid, Cetyltrimethylammoniumchlorid, Quaternium-83, Quaternium-87, Quaternium-22, Behenylamidopropyl-2,3-dihydroxypropyldimethylammoniumchlorid, Palmitylamidopropyltrimethylammoniumchlorid und Stearamidopropyldimethylamin.

## Revendications

1. Procédé de déformation permanente de fibres de kératine, comprenant les étapes suivantes :
soumission des fibres de kératine à une tension mécanique, application sur les fibres de kératine d'une composition comprenant au moins un tensioactif cationique en une quantité de 0,01 à 15 % en poids par rapport au poids total de la composition ; puis placement des fibres de kératine dans un espace occlusif pour limiter l'évaporation des composants évaporables dans la composition ; et ensuite chauffage des fibres de kératine à une température située dans la plage allant de 60 à 200°C,
dans lequel la composition ne contient ni agent réducteur ni source d'ions carbonate de formule : 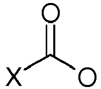 dans laquelle X est un groupe choisi dans l'ensemble constitué par O⁻, OH, NH₂, O-OH et O-COO⁻,
ledit procédé comprenant en outre l'étape de rinçage des fibres de kératine après l'étape d'application de la composition sur les fibres de kératine et/ou après l'étape de chauffage des fibres de kératine.

2. Procédé selon la revendication 1, dans lequel l'étape de soumission des fibres de kératine à une tension mécanique est effectuée après l'étape d'application sur les fibres de kératine de la composition comprenant au moins un tensioactif cationique.

3. Procédé selon la revendication 1 ou 2, dans lequel l'espace occlusif est formé par au moins un moyen de revêtement.

4. Procédé selon la revendication 3, dans lequel le moyen de revêtement est rigide ou flexible.

5. Procédé selon la revendication 3 ou 4, dans lequel le moyen de revêtement comprend au moins un élément choisi dans l'ensemble constitué par un film et une feuille.

6. Procédé selon l'une quelconque des revendications 1 à 5, dans lequel les fibres de kératine sont chauffées à une température de 60°C à 150°C durant l'étape de chauffage des fibres de kératine.

7. Procédé selon l'une quelconque des revendications 1 à 6, dans lequel les fibres de kératine sont chauffées par au moins un dispositif de chauffage délivrant au moins l'un choisi dans l'ensemble constitué par de l'air chaud, de la vapeur chaude, un chauffage par induction haute fréquence, un chauffage par micro-ondes, une irradiation de rayons infrarouges, un laser, et une irradiation par lampe flash.

8. Procédé selon la revendication 7, dans lequel le moyen de revêtement comprend le dispositif de chauffage.

9. Procédé selon l'une quelconque des revendications 1 à 8, dans lequel le tensioactif cationique est choisi dans l'ensemble constitué par les amines grasses primaires, secondaires et tertiaires, éventuellement polyoxyalkylénées, les sels d'ammonium quaternaire, et leurs mélanges.

10. Procédé selon la revendication 9, dans lequel les sels d'ammonium quaternaire sont choisis parmi :
ceux de formule générale (V) ci-dessous : 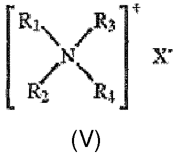 dans laquelle R₁, R₂, R₃ et R₄, qui peuvent être identiques ou différents, sont choisis parmi les radicaux aliphatiques linéaires et ramifiés comprenant de 1 à 30 atomes de carbone, et les radicaux aromatiques ; et X⁻ est choisi parmi les halogénures, les phosphates, les acétates, les lactates, les alkylsulfates en C₂ à C₆, et les alkyl- ou alkylaryl-sulfonates ;
les sels d'ammonium quaternaire d'imidazoline ;
les sels d'ammonium diquaternaire de formule (VII) : 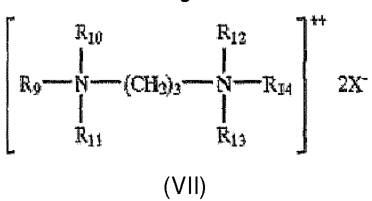 dans laquelle R₉ est choisi parmi les radicaux aliphatiques comprenant de 16 à 30 atomes de carbone ; R₁₀, R₁₁, R₁₂, R₁₃ et R₁₄, qui peuvent être identiques ou différents, sont choisis parmi l'hydrogène et les radicaux alkyle comprenant de 1 à 4 atomes de carbone ; et X⁻ est choisi parmi les halogénures, les acétates, les phosphates, les nitrates, les éthylsulfates, et les méthylsulfates ; et
les sels d'ammonium quaternaire comprenant au moins une fonction ester.

11. Procédé selon la revendication 10, dans lequel les sels d'ammonium quaternaire d'imidazoline sont choisis parmi ceux de formule (VI) ci-dessous : 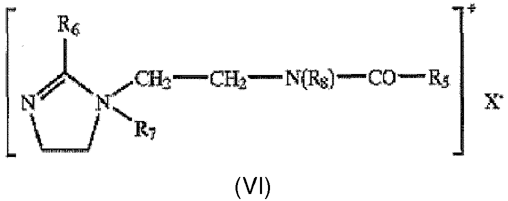 dans laquelle R₅ est choisi parmi les radicaux alcényle et alkyle comprenant de 8 à 30 atomes de carbone ; R₆ est choisi parmi l'hydrogène, les radicaux alkyle en C₁ à C₄, et les radicaux alcényle et alkyle comprenant de 8 à 30 atomes de carbone ; R₇ est choisi parmi les radicaux alkyle en C₁ à C₄ ; R₈ est choisi parmi l'hydrogène et les radicaux alkyle en C₁ à C₄ ; et X⁻ est choisi parmi les halogénures, les phosphates, les acétates, les lactates, les alkylsulfates, les alkylsulfonates, et les alkylarylsulfonates.

12. Procédé selon l'une quelconque des revendications 1 à 11, dans lequel le tensioactif cationique est choisi dans l'ensemble constitué par le chlorure de béhényltriméthylammonium, le chlorure de cétyltriméthylammonium, le quaternium-83, le quaternium-87, le quaternium-22, le chlorure de béhénylamidopropyl-2,3-dihydroxypropyldiméthylammonium, le chlorure de palmitylamidopropyltriméthylammonium, et la stéaramidopropyldiméthylamine.
